# EUROPÄISCHE PATENTSCHRIFT

(11) **EP 2 282 987 B1**
(45) Veröffentlichungstag und Bekanntmachung des Hinweises auf die Patenterteilung: **13.08.2014**
(21) Anmeldenummer: 09753749.2
(22) Anmeldetag: 17.04.2009
(51) Int. Cl.: C07C 51/43, C07C 51/44, C07C 51/353, C07C 57/13

(54) **VERFAHREN ZUR HERSTELLUNG VON UNGESÄTTIGTEN DICARBONSÄUREN AUS CYCLOALKENEN UND ACRYLSÄURE MITTELS METATHESE**
PROCESS FOR THE SYNTHESIS OF UNSATURATED DICARBOXYLIC ACIDS FROM CYCLOALKENES AND ACRYLIC ACID BY WAY OF METATHESIS
PROCÉDÉ DE SYNTHÈSE D'ACIDES DICARBOXYLIQUES INSATURES PRODUITS PAR METATHESE A PARTIR DE CYCLOALCÈNES ET D'ACIDE ACRYLIQUE

(30) Priorität: 30.05.2008 DE 102008002090
(43) Veröffentlichungstag der Anmeldung: 16.02.2011
(73) Patentinhaber: Evonik Degussa GmbH, 45128 Essen (DE)
(72) Erfinder: HANNEN, Peter, 45659 Recklinghausen (DE); ROOS, Martin, 45721 Haltern am See (DE); HÄGER, Harald, 59348 Lüdinghausen (DE)
(86) Internationale Anmeldenummer: PCT/EP2009/054599
(87) Internationale Veröffentlichungsnummer: WO 2009/144094

(56) Entgegenhaltungen:
- WO-A-02/079127
- SAITO I ET AL: "Synthesis of Synthetic alpha,omega-Dicarboxylic Acids and Unsaturated Carboxylic Acids from Silyl Enol Ethers" TETRAHEDRON LETTERS, Bd. 24, Nr. 41, 1. Januar 1983 (1983-01-01), Seiten 4439-4442, XP002529873 ISSN: 0040-4039
- ENGLISH, JAMES, JR.: "Wound hormones of plants. V. The synthesis of some analogs of traumatic acid" JOURNAL OF THE AMERICAN CHEMICAL SOCIETY, Bd. 63, 1941, Seiten 941-943, XP002551611

## Beschreibung

Aus cyclischen Kohlenwasserstoffen wie Cyclooctadien (COD), Cyclododecen (CDEN), Cyclododecatrien (CDT), Cyclohepten, Cyclohexen und Cyclopenten lassen sich über eine Metathese-Reaktion mittels eines geeigneten Katalysators α,β-ungesättigte Dicarbonsäuren herstellen **(Schema 1).**

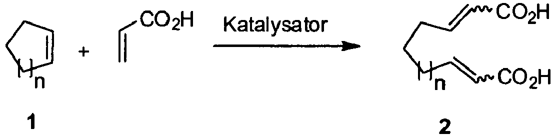

**Schema 1:** Allgemeine Darstellung zur Herstellung ungesättigter Dicarbonsäuren (2) aus cyclischen ungesättigten Kohlenwasserstoffen (1) über Metathese mit Acrylsäure.

Aus den erhaltenen α,β-ungesättigten Dicarbonsäuren lassen sich durch Polykondensation mit Diaminen oder Diolen entsprechend Polyamide bzw. Polyester gewinnen. Ein besonderes Merkmal dieser Kunststoffe ist die Möglichkeit der Quervernetzung über die Doppelbindungen. Über eine Hydrierung kommt man in einem Schritt zu den entsprechenden gesättigten Dicarbonsäuren. Des Weiteren lassen sich diese Verbindungen ohne größeren Aufwand zu den entsprechenden Diolen und Diaminen umsetzten. Neben den Polyestern ließen sich die Diole auch zur Herstellung von Polyurethanen verwenden.

### Stand der Technik:

Das hier beschriebene Verfahren stellt eine erhebliche Verbesserung zu herkömmlichen Verfahren dar.
***(***Morgan, John, P., Morrill, Christie; Grubbs, Robert, H.; Choi, Tae-Lim WO 02/079127 A1***.*** Choi, T-L.; Lee, C. W.; Chatterjee, A. K.; Grubbs, R. H. J. Am. Chem. Soc. 2001,123, 10417 10418***.***
Randl, S.; Connon, S. J.; Blechert, S. J. Chem. Soc., Chem. Commun. 2001,1796-1797***.)***

WO 02/079127 A beschreibt ein Verfahren zur Herstellung von ungesättigten Dicarbonsäuren mittels der ringöffnenden Krezsmetathese (ROCM) aus cyclischen Olefinen und α,β-ungesättigten Carbonsäuren in Gegenwart von Rutheniumkatalysatoren.

Bisher musste bei der oben beschriebenen Umsetzung in hoher Verdünnung gearbeitet werden um die als Konkurrenzreaktion ablaufende Oligomerisierung bzw. Polymerisation zu unterbinden. Zudem kommt überwiegend Dichlormethan als Lösungsmittel zum Einsatz, das aufgrund seines gesundheitsgefährdenden Potentials für eine technische Umsetzung von Nachteil ist.

Es wurde nun überraschenderweise gefunden, dass es durch ein Verfahren gemäß den Ansprüchen möglich ist, das Gleichgewicht vollständig in Richtung des gewünschten Produktes zu verschieben ohne in großer Verdünnung zu arbeiten. Zudem wird durch das beschriebene Verfahren ein effektives Recyceln des Katalysators möglich. Dies wird erreicht indem man, im Gegensatz zur bisherigen Praxis, bei Substratkonzentrationen von > 1 Mol/L des ungesättigten cyclischenKohlenwasserstoffs arbeitet. Im Laufe der Reaktion fällt bei Überschreiten des Löslichkeitsproduktes die α,β-ungesättigte Dicarbonsäure aus und wird somit dem Gleichgewicht (in der homogenen Phase) entzogen.

Ein entscheidender Vorteil der hier beschriebenen Erfindung ist also die Tatsache, dass das Produkt bei der Reaktion als Feststoff ausfällt. Da es sich bei der Ring-öffnenden Kreuzmetathese um eine Gleichgewichtsreaktion handelt besteht so die Möglichkeit- zusätzlich zur Freisetzung von Ethylen- das Gleichgewicht auf die Seite des gewünschten Produktes zu schieben. Auch wenn der Umsatz zu einem Zeitpunkt x nicht vollständig ist bzw. das Produkt zu einem Teil in Lösung bleibt, kann durch eine kontinuierliche Fahrweise letztendlich ein vollständiger Umsatz an Edukt erreicht werden.

Neben der effektiven Beeinflussung des Reaktionsgleichgewichtes zugunsten des Produktes ist die leichte Abtrennung der Produkte und damit einhergehend die Rückgewinnung des Katalysators eine große Erleichterung in der Prozessführung. Der eingesetzte Katalysator bleibt in Lösung und kann recycelt werden.

In bisherigen Verfahren muss das Reaktionsgemisch als Ganzes aufgearbeitet werden. Dies geschieht indem das Lösungsmittel abdestilliert und der Rückstand chromatographisch gereinigt wird. Besonders die chromatographische Aufreinigung des Rohproduktes erfordert den Einsatz großer Mengen an Lösungsmittel und Energie zum Entfernen desselbigen, was für eine technische Umsetzung unpraktikabel ist.

Die beschriebene Reaktion wird bei Temperaturen von 10 bis 100 °C, bevorzugt bei 20 bis 80 °C und besonders bevorzugt bei 20 bis 60 °C durchgeführt.

Die beschriebe Reaktion wird unter Verwendung eines Lösungsmittels durchgeführt. Als Lösungsmittel eignen sich acyclische als auch cyclische Kohlenwasserstoffe. Besonders eigenen sich aromatische halogenierte Kohlenwasserstoffe und ganz besonders eignen sich Aromaten mit Alkygruppen.

Bei Durchführung in Lösung sind Konzentrationen von 1 bis 2 M bevorzugt und besonders bevorzugt sind Konzentrationen von 2 bis 4 M an Cycloalken, bezogen auf das Lösungsmittel.

Bei dem beschriebenen Verfahren wird der Katalysator bezogen auf die Menge an ungesättigtem Cycloalken in Mengen von 5 bis 0,0001 mol-% eingesetzt. Bevorzugt sind Mengen von 2 bis 0,001 mol-% und besonders bevorzugt sind Mengen von 1 bis 0,5 mol-% Katalysator bezogen auf die Stoffmenge an eingesetztem ungesättigten Cycloalken.

Um die α,β-ungesättigte Dicarbonsäure in Polymergrade-Qualität zu erhalten, bietet sich die Aufreinigung über Kristallisation, Destillation oder eine Kombination von beiden an.

Als Katalysatoren eignen sich Ruthenium-carben Komplexe, die als eines der charakteristischen Merkmale einen N-heterocyclischen-Carben-Liganden tragen. Beispiele bevorzugter Katalysatoren finden sich in Bild 1. Besonders bevorzugt sind dabei Katalysatoren des Typs 7, mit einer Elektronen-ziehenden Gruppe R' am Benzylidenliganden.

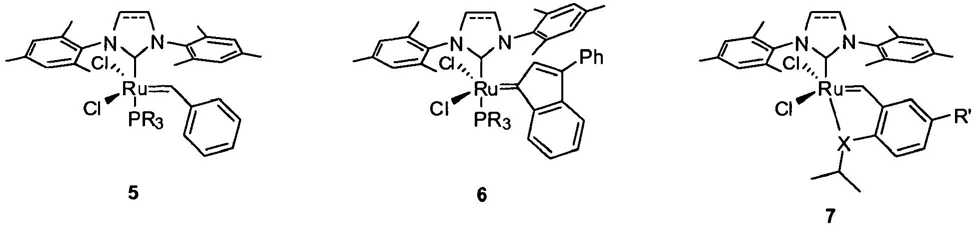

### Beispiele:

### Vergleichsbeispiel

1. 1,3-Bis(2,4,6-trimethylphenyl)-4,5-dihydroimidazol-2-yliden[2-(i-propoxy)-5-(N,N-dimethylaminosulfonyl)phenyl]methylen Ruthenium (II) dichlorid (1 mol-% bezogen auf das Cycloalken) wird in einem Schlenk-Gefäß in Toluol (2,25 mL) unter Argon vorgelegt. Eine Lösung von Cyclopenten (0,3 g, 4,4 mmol) und Acrylsäure (0,79 g, 11 mmol) in Toluol (2,25 mL) werden zur Katalysator-Lösung zugetropft. Das Reaktionsgemisch wird eine Stunde bei 60 °C gerührt und im Anschluss auf Raumtemperatur abgekühlt. Der sich abscheidende Feststoff wird abfiltriert, mit wenig kaltem Toluol gewaschen und im Vakuum getrocknet. Das Produkt wurde als weißer Feststoff erhalten (0,128 g, 16 %). Mittels NMR-Analytik wurde eine Reinheit von 98,9 % bestimmt.

### Erfindungsgemäße Beispiele

2. 1,3-Bis(2,4,6-trimethylphenyl)-4,5-dihydroimidazol-2-yliden[2-(i-propoxy)-5-(N,N-dimethylaminosulfonyl)phenyl]methylen Ruthenium (II) dichlorid (1 mol-% bezogen auf das Cycloalken) wird in einem Schlenk-Gefäß in Toluol (1,75 mL) unter Argon vorgelegt. Eine Lösung von Cyclohexen (0,3 g, 3,65 mmol) und Acrylsäure (0,66 g, 9,13 mmol) in Toluol (1,75 mL) werden zur Katalysator-Lösung zugetropft. Das Reaktionsgemisch wird eine Stunde bei 60 °C gerührt und im Anschluss auf Raumtemperatur abgekühlt. Der sich abscheidende Feststoff wird abfiltriert, mit wenig kaltem Toluol gewaschen und im Vakuum getrocknet. Das Produkt wurde als weißer Feststoff erhalten (0,272 g, 38 %). Mittels NMR-Analytik wurde eine Reinheit von 99,2 % bestimmt.
3. 1,3-Bis(2,4,6-trimethylphenyl)-4,5-dihydroimidazol-2-yliden[2-(i-propoxy)-5-(N,N-dimethylaminosulfonyl)phenyl]methylen Ruthenium (II) dichlorid (1 mol-% bezogen auf das Cycloalken) wird in einem Schlenk-Gefäß in Toluol (1,50 mL) unter Argon vorgelegt. Eine Lösung von Cyclohepten (0,3 g, 3,12 mmol) und Acrylsäure (0,56 g, 7,80 mmol) in Toluol (1,50 mL) werden zur Katalysator-Lösung zugetropft. Das Reaktionsgemisch wird eine Stunde bei 60 °C gerührt und im Anschluss auf Raumtemperatur abgekühlt. Der sich abscheidende Feststoff wird abfiltriert, mit wenig kaltem Toluol gewaschen und im Vakuum getrocknet. Das Produkt wurde als weißer Feststoff erhalten (0,223 g, 14 %). Mittels NMR-Analytik wurde eine Reinheit von 91 % bestimmt.
4. 1,3-Bis(2,4,6-trimethylphenyl)-4,5-dihydroimidazol-2-yliden[2-(i-propoxy)-5-(N,N-dimethylaminosulfonyl)phenyl]methylen Ruthenium (II) dichlorid (1 mol-% bezogen auf das Cycloalken) wird in einem Schlenk-Gefäß in Toluol (1,80 mL) unter Argon vorgelegt. Eine Lösung von Cycloctadien (0,4 g, 3,70 mmol) und Acrylsäure (1,33 g, 18,49 mmol) in Toluol (1,80 mL) werden zur Katalysator-Lösung zugetropft. Das Reaktionsgemisch wird eine Stunde bei 60 °C gerührt und im Anschluss auf Raumtemperatur abgekühlt. Der sich abscheidende Feststoff wird abfiltriert, mit wenig kaltem Toluol gewaschen und im Vakuum getrocknet. Das Produkt wurde als weißer Feststoff erhalten (0,158 g, 25 %). Mittels HPLC-Analytik wurde eine Reinheit von 98 % bestimmt.

## Patentansprüche

1. Verfahren zur Herstellung von ungesättigten Dicarbonsäuren,
**dadurch gekennzeichnet,**
**dass** ungesättigte, cyclische Kohlenwasserstoffe und Acrylsäure mit einem Ruthenium-Katalysator über eine Metathese-Reaktion bei Substratkonzentrationen von > 1 Mol/L des ungesättigten cyclischen Kohlenwasserstoffs umgesetzt werden, wobei die dabei entstehende Dicarbonsäure bei Überschreitung des Löslichkeitsproduktes ausfällt.

2. Verfahren nach Anspruch 1,
**dadurch gekennzeichnet,**
**dass** als ungesättigte cyclische Kohlenwasserstoffe Cyclooctadien, Cyclododecen, Cyclododecatrien, Cyclohepten, Cyclohexen oder Cyclopenten eingesetzt werden

3. Verfahren nach Anspruch 1,
**dadurch gekennzeichnet,**
**dass** bei der Reaktionsdurchführung in Lösung der ungesättigte cyclische Kohlenwasserstoff in Konzentrationen von 2 bis 4 Mol/L vorliegt.

4. Verfahren nach einem der vorhergehenden Ansprüche,
**dadurch gekennzeichnet,**
**dass** als Katalysatoren Ruthenium-carben Komplexe, die einen N-heterocyclischen Liganden tragen eingesetzt werden.

5. Verfahren nach einem der vorhergehenden Ansprüche,
**dadurch gekennzeichnet,**
**dass** die Ruthenium-Katalysatoren in Mengen von 5 bis 0,0001 mol-%, bezogen auf die Stoffmenge an ungesättigtem cyclischen Kohlenwasserstoff, eingesetzt werden.

6. Verfahren nach einem der vorhergehenden Ansprüche,
**dadurch gekennzeichnet,**
**dass** die Ruthenium-Katalysatoren in Mengen von 2 bis 0,001 mol-%, bezogen auf die Stoffmenge an ungesättigtem cyclischen Kohlenwasserstoff, eingesetzt werden.

7. Verfahren nach einem der vorhergehenden Ansprüche,
**dadurch gekennzeichnet,**
**dass** die Ruthenium-Katalysatoren in Mengen von 1 bis 0,5 mol-%, bezogen auf die Stoffmenge an ungesättigtem cyclischen Kohlenwasserstoff, eingesetzt werden.

8. Verfahren nach einem der vorhergehenden Ansprüche,
**dadurch gekennzeichnet,**
**dass** als Lösungsmittel acyclische als auch cyclische Kohlenwasserstoffe eingesetzt werden.

9. Verfahren nach einem der vorhergehenden Ansprüche,
**dadurch gekennzeichnet,**
**dass** als Lösungsmittel aromatische halogenierte Kohlenwasserstoffe eingesetzt werden.

10. Verfahren nach einem der vorhergehenden Ansprüche,
**dadurch gekennzeichnet,**
**dass** die erhaltene ungesättigte Dicarbonsäure in einem zweiten Schritt hydriert wird.

11. Verfahren nach einem der vorhergehenden Ansprüche,
**dadurch gekennzeichnet,**
**dass** die im Filtrat gelösten Ruthenium-Katalysatoren recycelt werden.

12. Verfahren nach einem der vorhergehenden Ansprüche,
**dadurch gekennzeichnet,**
**dass** die erhaltene ungesättigte Dicarbonsäure über Kristallisation, Destillation oder eine Kombination von beiden aufgereinigt wird.

## Claims

1. Process for preparing unsaturated dicarboxylic acids,
**characterized in that**
unsaturated, cyclic hydrocarbons and acrylic acid are reacted by means of a metathesis reaction at substrate concentrations of > 1 mol/l of the unsaturated cyclic hydrocarbon with a ruthenium catalyst, with the dicarboxylic acid formed precipitating using the solubility product is exceeded.

2. Process according to Claim 1,
**characterized in that**
cyclooctadiene, cyclododecene, cyclododecatriene, cycloheptene, cyclohexene or cyclopentene are used as unsaturated cyclic hydrocarbons.

3. Process according to Claim 1,
**characterized in that**
when the reaction is carried out in solution, the unsaturated cyclic hydrocarbon is present in concentrations of from 2 to 4 mol/l.

4. Process according to any of the preceding claims, **characterized in that**
ruthenium-carbene complexes which bear an N-heterocyclic ligand are used as catalysts.

5. Process according to any of the preceding claims,
**characterized in that**
the ruthenium catalysts are used in amounts of from 5 to 0.0001 mol%, based on the molar amount of unsaturated cyclic hydrocarbon.

6. Process according to any of the preceding claims,
**characterized in that**
the ruthenium catalysts are used in amounts of from 2 to 0.001 mol%, based on the molar amount of unsaturated cyclic hydrocarbon.

7. Process according to any of the preceding claims,
**characterized in that**
the ruthenium catalysts are used in amounts of from 1 to 0.5 mol%, based on the molar amount of unsaturated cyclic hydrocarbon.

8. Process according to any of the preceding claims,
**characterized in that**
acyclic or cyclic hydrocarbons used as solvents.

9. Process according to any of the preceding claims,
**characterized in that**
aromatic halogenated hydrocarbons are used as solvents.

10. Process according to any of the preceding claims,
**characterized in that**
the unsaturated dicarboxylic acid obtained is hydrogenated in a second step.

11. Process according to any of the preceding claims,
**characterized in that**
the ruthenium catalysts dissolved in the filtrate are recycled.

12. Process according to any of the preceding claims,
**characterized in that**
the unsaturated dicarboxylic acid obtained is purified by crystallization, distillation or a combination of the two.

## Revendications

1. Procédé pour la préparation d'acides dicarboxyliques insaturés, **caractérisé en ce qu'**on fait réagir des hydrocarbures cycliques insaturés et de l'acide acrylique avec un catalyseur à base de ruthénium via une réaction de métathèse à des concentrations en substrat > 1 mole/l d'hydrocarbure cyclique insaturé, l'acide dicarboxylique qui se forme précipitant lors du dépassement du produit de solubilité.

2. Procédé selon la revendication 1, **caractérisé en ce qu'**on utilise, comme hydrocarbures cycliques insaturés, du cyclooctadiène, du cyclododécène, du cyclododécatriène, du cycloheptène, du cyclohexène ou du cyclopentène.

3. Procédé selon la revendication 1, **caractérisé en ce que** lors de la réalisation de la réaction en solution, l'hydrocarbure cyclique insaturé se trouve en des concentrations de 2 à 4 moles/l.

4. Procédé selon l'une quelconque des revendications précédentes, **caractérisé en ce qu'**on utilise, comme catalyseurs, des complexes de ruthénium-carbène qui portent un ligand N-hétérocyclique.

5. Procédé selon l'une quelconque des revendications précédentes, **caractérisé en ce que** les catalyseurs à base de ruthénium sont utilisés en des quantités de 5 à 0,0001% en mole par rapport à la quantité de substance d'hydrocarbure cyclique insaturé.

6. Procédé selon l'une quelconque des revendications précédentes, **caractérisé en ce que** les catalyseurs à base de ruthénium sont utilisés en des quantités de 2 à 0,001% en mole par rapport à la quantité de substance d'hydrocarbure cyclique insaturé.

7. Procédé selon l'une quelconque des revendications précédentes, **caractérisé en ce que** les catalyseurs à base de ruthénium sont utilisés en des quantités de 1 à 0,5% en mole par rapport à la quantité de substance d'hydrocarbure cyclique insaturé.

8. Procédé selon l'une quelconque des revendications précédentes, **caractérisé en ce qu'**on utilise, comme solvants, des hydrocarbures acycliques ainsi que cycliques.

9. Procédé selon l'une quelconque des revendications précédentes, **caractérisé en ce qu'**on utilise, comme solvants, des hydrocarbures aromatiques halogénés.

10. Procédé selon l'une quelconque des revendications précédentes, **caractérisé en ce que** l'acide dicarboxylique insaturé obtenu est hydrogéné dans une deuxième étape.

11. Procédé selon l'une quelconque des revendications précédentes, **caractérisé en ce que** les catalyseurs à base de ruthénium dissous dans le filtrat sont recyclés.

12. Procédé selon l'une quelconque des revendications précédentes, **caractérisé en ce que** l'acide dicarboxylique insaturé obtenu est purifié par cristallisation, distillation ou une combinaison des deux.
